# EUROPEAN PATENT SPECIFICATION

(11) **EP 0 363 192 B1**
(45) Date of publication and mention of the grant of the patent: **30.08.1995**
(21) Application number: 89310185.7
(22) Date of filing: 05.10.1989
(51) Int. Cl.: C07K 1/06, C07K 1/12

(54) **A process for the formation of intramolecular disulfide bridge in a polypeptide**
Verfahren zur Herstellung einer intramolekularen Disulphid-Brücke in Polypeptiden
Procédé pour la formation du pont disulfure intramoléculaire dans les polypeptides

(30) Priority: 07.10.1988 JP 254530/88
(43) Date of publication of application: 11.04.1990
(73) Proprietor: FUJISAWA PHARMACEUTICAL CO., LTD., Osaka-shi Osaka 541 (JP)
(72) Inventor: Niwa, Mineo, Muko-shi Kyoto 617 (JP); Kobayashi, Masakazu, Takarazuka-shi Hyogo 665 (JP); Ishii, Yoshinori, Suita-shi Osaka 565 (JP); Ueda, Ikuo, Toyonaka-shi Osaka 560 (JP)
(74) Representative: Gaunt, Robert John

(56) References cited:
- US-A- 3 929 758
- US-A- 4 216 141
- CHEMICAL ABSTRACTS, vol. 66, no. 4, 23rd January 1967, page 1588, abstract no.16538e, Columbus, Ohio, US; C. LITTLE et al.: "Effect of oxidizing agents on aprotein sulfhydryl group", & BIOCHEM. J. 101(2), 11P-12P(1966)

## Description

This invention relates to a process for the formation of an intramolecular disulfide bridge in a polypeptide. More particularly, it relates to a process for the formation of an intramolecular disulfide bridge in a cysteine-containing polypeptide through the use of hydrogen peroxide.

There are several known processes for the formation of an intramolecular disulfide bridge between two cysteine residues in a polypeptide. Such processes include the air-oxidation process [see B.B.R.C. 119, 131 (1984)], the iodine-oxidation process [see "Peptide Chemistry" 229-234 (1984)], and the potassium ferricyanide-oxidation process [see "Peptide Chemistry" 241-246 (1984)].

These known processes suffer from some disadvantages, however, as follows. In the air-oxidation process, the speed at which the reaction proceeds is very slow and, in particular, it hardly proceeds at all under denaturing conditions (such as in a highly concentrated salt or urea-aqueous solution of a polypeptide). In the iodine-oxidation process, tyrosine residue(s) present in a polypeptide may be disadvantageously iodinated. While in the potassium ferricyanide-oxidation process, there is the problem of environmental pollution since the resultant waste water contains CN⁻.

It is well known to use hydrogen peroxide as an oxidising agent for an organic compound. In the case of polypeptides, however, it is generally recognised that hydrogen peroxide does not oxidize mercapto groups in polypeptides (such as enzymes) to produce intramolecular disulfide bridges [European J. Biochem. 10 (1969) 533-538]. Further, it is widely believed that methionine residue(s), which may co-exist in a cysteine-containing polypeptide, will be surely oxidized to give methionine sulfoxide residue(s) if such a polypeptide is forced to be oxidized with hydrogen peroxide. For these reasons, a hydrogen peroxide based oxidation process is considered as being an inappropriate means to produce biologically active polypeptides.

Accordingly, an object of the present invention is to provide a process which easily and reliably gives the desired biologically active polypeptide by forming an intramolecular disulfide bridge in a cysteine-containing polypeptide through the use of hydrogen peroxide, even though one or more methionine residues may be present in the polypeptide as well.

The inventors of the present invention have studied to establish the new process to form an intramolecular disulfide bridge by oxidizing a cysteine-containing polypeptide, and which is prepared through recombinant DNA technology or by organic synthesis.

In the present invention, the methionine residue(s) which may co-exist in the cysteine-containing polypeptide is not (or not significantly) oxidized, contrary to the general expectation, during the course of the process to form an intramolecular disulfide bridge by oxidizing two cysteine residues using hydrogen peroxide.

The process of this invention is carried out by oxidizing a cysteine-containing polypeptide, or a cysteine- and methionine-containing polypeptide, with hydrogen peroxide to produce efficiently a biologically active polypeptide which has an intramolecular disulfide bond.

Cysteine-containing polypeptides or both cysteine- and methionine-containing polypeptides for use with the present invention include the so-called reduced form of polypeptide and which requires the formation of an intramolecular disulfide bridge in order to recover its biological activity. Such polypeptides may be prepared through recombinant DNA technology or by organic synthesis. Representative examples of the polypeptides include the reduced form of α-hANP (human atrial natriuretic peptide), the reduced form of somatostatin, the reduced form of IL-2 (interleukin-2), and the reduced form of TNF (tumour necrosis factor).

The reaction upon which the process of the present invention is based is carried out at a pH of from 6 to 11, and more preferably at around pH 8 to 11. The molar ratio of the hydrogen peroxide and the polypeptide is at least stoichiometric and is selected to be within the range of from 1:1 to 100:1, preferably from 1:1 to 3:1.

The reaction of the present invention is preferably carried out in the solution wherein the polypeptide is dissolved in suitable buffer solution. The reaction is usually carried out at ambient temperature.

The following examples are given for the purpose of illustrating the present invention in more detail.

### Example 1

Reduced form of α-hANP (10 mg) was dissolved in 10 mM sodium phosphate buffer solution (9.3 ml, pH 8.5) containing 0.5 M sodium chloride, and 10 mM aqueous hydrogen peroxide (0.66 ml) was added thereto. The reaction mixture was allowed to stand for 10 minutes at ambient temperature. After the reaction was over, the oxidized form of α-hANP (the natural form of α-hANP containing a disulfide bond) in the reaction mixture was quantitatively determined with reversed phase high pressure liquid chromatography (RP-HPLC). The yield of the oxidized form of α-hANP was 9.6 mg (96%) and that of α-hANP wherein methionine residue was also oxidized, [Met (0)-α-hANP], was only 0.15 mg.

### Comparative Example 1

Reduced form of α-hANP (110 mg) was dissolved in 10 mM sodium phosphate buffer (50 ml, pH 8.5) containing 0.5 M sodium chloride. The reaction mixture was allowed to stand overnight at ambient temperature, and then the resultant oxidized form of α-hANP was quantitatively determined with RP-HPLC. The yield of the oxidized form of α-hANP was 33.8 mg (30.7%). The major part (68.6 mg) of the raw material was recovered as unchanged.

The experimental condition of RP-HPLC was as follows:-

| | |
|---|---|
| Column | YMC AP-302 ODS (⌀4.6 x 150mm) |
| Solvent | A 20% CH₃CN in 0.01 MTFA |
| | B 60% CH₃CN in 0.01 MTFA |
| Elution | A → B 40 minutes |
| Fluid Speed | 1.0 ml/min |
| Detection | 214 nm |

### Example 2

Reduced form of α-hANP (0.5 mg) was dissolved in 10 mM tris-HCl buffer (1 ml, pH 8.0) containing 6 M guanidine hydrochloride, and 10 mM aqueous hydrogen peroxide (60»l) was added thereto. The reaction mixture was allowed to stand for 10 minutes at ambient temperature. The reaction mixture was determined for RP-HPLC in the same manner as Example 1. The yield of the oxidized form of α-hANP was 0.45 mg (90%) and the yield of Met (0)-α-hANP was only 25»g.

Reduced form of α-hANP (0.5 mg) was dissolved in 10 mM tris-HCl buffer (1ml, pH 8.0) containing 6 M guanidine hydrochloride. The reaction mixture was allowed to stand overnight at ambient temperature. The reaction mixture was determined for RP-HPLC in the same manner as Example 1. The air-oxidation reaction did not fully proceed and the raw material was quantitatively recovered as unchanged.

From the above result, it is concluded that the intramolecular disulfide bridge is successively formed to produce the oxidized form of α-hANP (even under the condition of the highly concentrated salt solution wherein the previously known air-oxidation reaction does not proceed to completion).

### Example 3

Reduced form of somatostatin was oxidized with hydrogen peroxide in the same manner as Example 2 to produce the oxidized form of somatostatin (natural type somatostatin wherein an intramolecular disulfide bridge was formed). The yield was 93%.

## Claims

1. A process for the formation of an intramolecular disulfide bridge in a polypeptide which is characterized by reacting hydrogen peroxide with a cysteine-containing polypeptide to produce a biologically active polypeptide having the said intramolecular disulfide bridge, the reaction being carried out at a pH of from 6 to 11, and the molar ratio of hydrogen peroxide to polypeptide being within the range of from 1:1 to 100:1.

2. A process as claimed in claim 1, which is characterized by reacting the hydrogen peroxide with a cysteine- and methionine-containing polypeptide to produce the biologically active polypeptide having the said intramolecular disulfide bridge.

3. The process of claim 1, wherein the cysteine-containing polypeptide is somatostatin.

4. The process of claim 2, wherein the cysteine- and methionine-containing polypeptide is human atrial natriuretic polypeptide.

5. The process of any of the preceding claims, wherein the reaction is carried out at around pH 8 to 11.

## Patentansprüche

1. Verfahren zur Bildung einer intramolekularen Disulfidbrücke in einem Polypeptid, gekennzeichnet durch die Umsetzung von Wasserstoffperoxid mit einem Cystein enthaltenden Polypeptid unter Bildung eines biologisch aktiven Polypeptids, welches die genannte intramolekulare Disulfidbrücke aufweist, wobei die Reaktion bei einem pH-Wert von 6 bis 11 durchgeführt wird und das Molverhältnis zwischen Wasserstoffperoxid und Polypeptid innerhalb des Bereiches von 1:1 bis 100:1 liegt.

2. Verfahren nach Anspruch 1, gekennzeichnet durch die Umsetzung des Wasserstoffperoxids mit einem Cystein und Methionin enthaltenden Polypeptid unter Bildung des biologisch aktiven Polypeptids, welches die genannte intramolekulare Disulfidbrücke aufweist.

3. Verfahren nach Anspruch 1, worin das Cystein enthaltende Polypeptid Somatostatin ist.

4. Verfahren nach Anspruch 2, worin das Cystein und Methionin enthaltende Polypeptid ein atriales natriuretrisches Human-Polypeptid ist.

5. Verfahren nach einem der vorhergehenden Ansprüche, worin die Reaktion bei etwa pH 8 bis 11 durchgeführt wird.

## Revendications

1. Procédé de formation d'un pont disulfure intramoléculaire dans un polypeptide qui est caractérisé en ce qu'on fait réagir du peroxyde d'hydrogène avec un polypeptide contenant de la cystéine pour donner un polypeptide biologiquement actif ayant ce pont disulfure intramoléculaire, la réaction étant effectuée à un pH de 6 à 11, et le rapport molaire du peroxyde d'hydrogène au polypeptide étant dans l'intervalle de 1:1 à 100:1.

2. Procédé selon la revendication 1, qui est caractérisé en ce qu'on fait réagir le peroxyde d'hydrogène avec un polypeptide contenant de la cystéine et de la méthionine pour donner un polypeptide biologiquement actif ayant ce pont disulfure intramoléculaire.

3. Procedé selon la revendication 1, dans lequel le polypeptide contenant de la cystéine est la somatostatine.

4. Procédé selon la revendication 2, dans lequel le polypeptide contenant de la cystéine et dé la méthionine est un polypeptide natriurétique atrial humain.

5. Procédé selon l'une quelconque, des revendications précédentes, dans lequel la réaction est effectuée aux environs de pH 8 à 11.
